# EUROPEAN PATENT APPLICATION

(11) **EP 4 785 879 A1**
(43) Date of publication of application: **05.08.2026**
(21) Application number: 24870964.4
(22) Date of filing: 27.09.2024
(51) Int. Cl.: A61B 17/12

(54) **OCCLUSION DEVICE**

(30) Priority: 27.09.2023 CN 202322682209 U
(71) Applicant: Lifetech Scientific (Shenzhen) Co., Ltd., Shenzhen, Guangdong 518063 (CN)
(72) Inventor: LIU, Jianyong, Shenzhen, Guangdong 518063 (CN); FANG, Yu, Shenzhen, Guangdong 518063 (CN)
(74) Representative: Michalski Hüttermann & Partner mbB
(86) International application number: PCT/CN2024/121766
(87) International publication number: WO 2025/067427

(57) **Abstract**

An occlusion device (100). The occlusion device (100) includes a fixing portion (110, 310) and a sealing portion (120); the fixing portion (110, 310) includes a mesh braided structure; the fixing portion (110, 310) includes a central area (111, 311) and a plurality of edge areas (112, 312) surrounding the central area (111, 311) in a circumferential direction; and a recess (113, 3113) is formed between two adjacent edge areas (112, 312). Compared with the design of a traditional fixing portion (210) having a cylindrical outer edge, the fixing portion (110, 310) of the occlusion device (100) is provided with a plurality of recesses (113, 3113) and the edge areas (112, 312), which can be adaptively snapped into recesses (201) and protrusions (202) of an implantation area and abut against the surface of the implantation area, thereby expanding a contact area and obtaining a better anchoring capability.

## Description

### Technical Field

The present disclosure relates to the technical field of interventional medical devices, and particularly, to an occlusion device.

### Background Art

In recent years, among patients with non-valvular atrial fibrillation, 90% of stroke cases caused by atrial fibrillation originate from the left atrial appendage. Clinical data shows that during atrial fibrillation, surgical excision of the left atrial appendage in cardiac surgery can reduce the incidence of stroke in patients with atrial fibrillation, indicating the hazard of the left atrial appendage in thromboembolism. Since the left atrial appendage serves as a site for thrombus formation, occluding the orifice of the left atrial appendage can eliminate the basis for thrombus development in the left atrial appendage. Typically, occluding the left atrial appendage through a left atrial appendage occluder is an effective method for preventing stroke caused by atrial fibrillation.

To effectively occlude the left atrial appendage, the left atrial appendage occluder needs to be implanted in the left atrial appendage on a long-term basis to achieve the occlusion effect. Therefore, the left atrial appendage occluder must be equipped with a specific anchoring structure to ensure its long-term stable occlusion at the orifice and prevent it from dislodgement that may lead to device embolization and other problems.

A split occluder generally includes a fixing portion for fixing and a sealing portion for sealing. The fixing portion is usually anchored through sharp anchoring barbs or in other manners, without considering the tightness or status of contact between the fixing portion and the implant site. When the contact area between the fixing portion and the left atrial appendage is insufficient, a risk of dislodgement still exists, which cannot be improved in the prior art.

### Summary of the Disclosure

Based on this, for the problem that a contact area between a fixing portion and an implant site is insufficient in the existing left atrial appendage occluder, an improved occlusion device is provided. Details are as follows.

An occlusion device is provided, which includes a fixing portion and a sealing portion. The fixing portion includes a mesh braided structure; the fixing portion includes a central area and a plurality of edge areas surrounding the central area in a circumferential direction; and a recess is formed between two adjacent edge areas.

In one embodiment, the recess is closer to an axis of the occlusion device than a radial outer side of the edge area in a radial direction.

In one embodiment, a proximal end and a distal end of the fixing portion are converged and closed.

In one embodiment, the edge areas include wing portions extending outward, and two adjacent side faces between adjacent wing portions extend obliquely in the same direction on the same cross section.

In one embodiment, a number of edge areas of the fixing portion is between 4 and 6.

In one embodiment, the plurality of edge areas of the fixing portion are oblique and radial on an axial cross section.

In one embodiment, the plurality of edge areas of the fixing portion extend outward from a base in a clockwise direction, or the plurality of edge areas extend outward from a base in a counterclockwise direction.

In one embodiment, with a radial center line of the edge area as a dividing line, on an inner side of the edge area along the radial center line, a maximum separation distance between the adjacent wing portions is smaller than a maximum circumferential width of the adjacent wing portions.

In one embodiment, with a radial center line of the edge area as a dividing line, on the inner side of the edge area along the radial center line, a maximum separation distance between the adjacent wing portions is smaller than a maximum circumferential width of any wing portion.

In one embodiment, an anchoring component is arranged on the fixing portion, the anchoring component is bound to the fixing portion through a flexible component, and a free end of the anchoring component is exposed.

In one embodiment, the anchoring component includes a first hook and a second hook which are connected to each other, and the first hook is positioned on a distal side of the second hook.

Compared with the prior art, the present disclosure provides the occlusion device, which includes the fixing portion and the sealing portion, the fixing portion includes a mesh braided structure, the fixing portion includes the central area and the plurality of edge areas surrounding the central area in the circumferential direction, and the recess is formed between the two adjacent edge areas; and compared with the design of a traditional fixing portion having a cylindrical outer edge (i.e., a circular cross section), the fixing portion of the occlusion device in the present disclosure is provided with a plurality of recesses and the edge areas, which can be adaptively snap-fitted with protrusions and recesses of an implantation area and abut against the surface of the implantation area, thereby expanding the contact area and obtaining a better anchoring capability.

### Brief Description of the Drawings

FIG. 1 is a front view of an occlusion device in a natural state in Embodiment 1 of the present disclosure;
FIG. 2 is a top view of a fixing portion, from a distal end to a proximal end, of the occlusion device in Embodiment 1 of the present disclosure;
FIG. 3 is a schematic diagram of the fixing portion, in a deformed state after implantation, of the occlusion device in Embodiment 1 of the present disclosure;
FIG. 4 is a top view of a fixing portion, from a distal end to a proximal end, of a fixing portion of a traditional braided structure;
FIG. 5 is a schematic diagram of the fixing portion, in a deformed state after implantation, of the traditional braided structure;
FIG. 6 is a top view of an occlusion device from a distal end to a proximal end in Embodiment 2 of the present disclosure;
FIG. 7 is a schematic structural diagram of a fixing portion of an occlusion device in Embodiment 3 of the present disclosure;
FIG. 8 is a top view of the fixing portion, from a distal end to a proximal end, of the occlusion device in Embodiment 3 of the present disclosure;
FIG. 9 is a schematic structural diagram of a plurality of optional anchoring components in Embodiment 3 of the present disclosure;
FIG. 10 is a schematic structural diagram of an occlusion device in another Embodiment of the present disclosure; and
FIG. 11 is a top view of an occlusion device from a distal end to a proximal end in another Embodiment of the present disclosure.

### Detailed Description of the Disclosure

In order to make the objectives, technical solutions, and advantages of the present disclosure clearer, the following is a further detailed explanation of the present disclosure in conjunction with the accompanying drawings and embodiments. It should be understood that the described specific embodiments are only for explaining the present disclosure, rather than limiting the present disclosure.

It should be noted that in the field of interventional medical devices, one end, closer to an operator, of a medical device implanted in a human body or an animal is generally referred to as a "proximal end", and the other end farther from the operator is referred to as a "distal end". Based on this principle, a "proximal end" and a "distal end" of any component of the medical device are defined. An "axial direction" generally refers to a length direction of a medical device when the medical device is delivered, and a "radial direction" generally refers to a direction of the medical device, perpendicular to the "axial direction". Based on this principle, an "axial direction" and a "radial direction" of any component of the medical device are defined. "Connection" mentioned in the embodiments includes direct connection of two components or indirect connection of two components by means of other components.

The technical solutions in the present disclosure will be further elaborated below with reference to the specific embodiments.

### Embodiment 1

An occlusion device 100 provided by Embodiment 1 may be configured to occlude a left atrial appendage, or may be configured to occlude other body tissues with orifices, which include but not limited to an atrial septal defect, a patent ductus arteriosus, and the like. The occlusion device 100 will be described in details by taking an example of occluding the left atrial appendage.

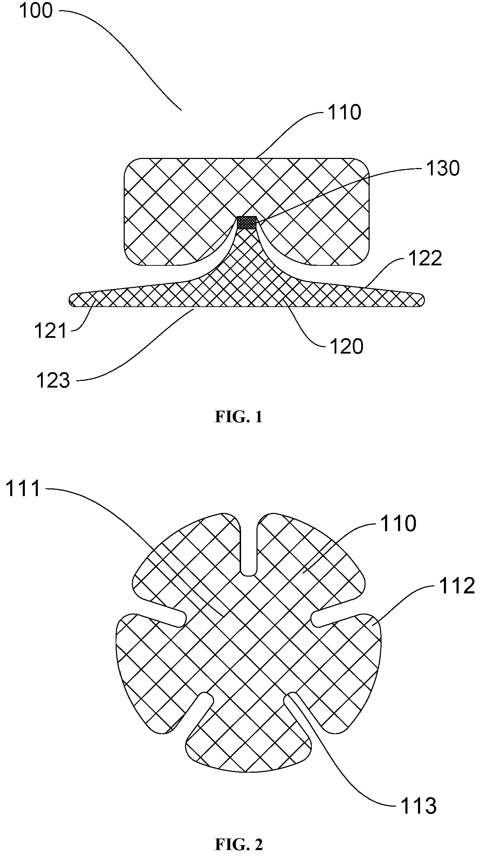

Referring to FIG. 1, FIG. 1 is a front view of the occlusion device 100 in a natural state in Embodiment 1 of the present disclosure. The occlusion device 100 includes a fixing portion 110 and a sealing portion 120 connected to the fixing portion 110. The sealing portion 120 and the fixing portion 110 are spaced in an axial direction of the occlusion device 100. The sealing portion 120 is positioned at a proximal end of the occlusion device 100, and the fixing portion 110 is positioned at a distal end of the occlusion device 100. The occlusion device 100 has a compressed state in which the occlusion device is accommodated in a sheath, to facilitate transport, and an unfolded state, as shown in FIG. 1, after the occlusion device extends from a distal end of the sheath and self-expands. A shape of the occlusion device 100 after it is released in a cavity of the left atrial appendage is identical to or substantially the same as that shown in FIG. 1. In other implementations, for example, when the occlusion device is configured to occlude an atrial septal defect, the sealing portion 120 and the fixing portion 110 may approach each other after release, to secure the occlusion device 100 to the septum between the left atrium and the right atrium.

In this embodiment, the fixing portion 110 is connected to a connecting component 130, and a distal end of the sealing portion 120 is converged and connected to the connecting component 130.

During manufacture of the sealing portion 120, a plurality of braiding wires 121 are braided to form a mesh tube, and two ends of the mesh tube close and secure end portions of the braiding wires 121 through a sleeve respectively. Then the mesh tube is heat-set into a disc shape, a cylindrical shape, or a plug shape, to obtain the sealing portion 120 configured to occlude the orifice of the left atrial appendage. The sealing portion 120 includes a distal disc surface 122 facing the fixing portion 110, and a proximal disc surface 123 opposite to the distal disc surface 122. Preferably, at least one layer of film body (not shown) serving as a flow-blocking film is arranged inside the sealing portion 120, the edge of the film body is secured to the braiding wires 121 at the edge of the sealing portion 120, and is generally secured through a suture, and the film body is between the distal disc surface 122 and the proximal disc surface 123. The film body is configured to prevent blood flow from one side of the sealing portion 120 to the other side, to prevent blood circulation between the left atrial appendage and the left atrium. In this embodiment, a distance between the distal disc surface 122 and the proximal disc surface 123 is not specified. Therefore, the sealing portion 120 is not specifically required to maintain a disc shape, and may also be a cylinder with a certain height.

Further, referring to FIG. 2, FIG. 2 is a top view of the fixing portion, from a distal end to a proximal end, of the occlusion device in Embodiment 1 of the present disclosure. In this embodiment, the fixing portion 110 is formed by braiding metal wires. Specifically, one or a plurality of metal wires are bent along a specific path, to form a mesh braided body, and the fixing portion 110 is allowed to have good adaptability to minimize damage to the left atrial appendage. It should be noted that compared with a rod-like structure formed by direct cutting, the braided structure provided in this embodiment exhibits higher adaptability for the following reasons. The diameter of the metal wires selected for braiding is small enough; otherwise, the metal wires cannot be processed to form the braided body; accordingly, if the rod-like structure adopts the same diameter as the braided structure, radial support force of the rod-like structure against deformation of the left atrial appendage is extremely small, and this prevents the rod-like fixing portion 110 from achieving a fixing purpose, leading to dislodgement of the entire occlusion device 100. In addition, the metal wires are pre-bent in a braiding process, applying force in the pre-bending direction more easily deforms the metal wires, and the braided body just leverages this characteristic by transferring deformation of a metal wire at a specific position to adjacent and interwoven metal wires, thereby dispersing concentrated stress. As a result, force applied to a certain point of the braided body is effectively distributed throughout the entire structure along the braided structure.

For this embodiment, in addition to having the braided structure, the fixing portion 110 includes a central area 111 and a plurality of edge areas 112 surrounding the central area 111; a cross-sectional shape of the central area 111 is approximately circular; and for the central area 111, one side of the edge area 112 extends from the central area 111, projects radially outward, and then curves back toward the central area 111. In other words, in this embodiment, the fixing portion 110 includes a plurality of edge areas 112 distributed around the central area 111 and projecting radially outward. In this embodiment, the edge areas 112 are distributed radially, a recess 113 is formed between two adjacent edge areas 112, and in terms of location, the recess 113 is closer to an axis than a radial outer side of the edge area 112 in a radial direction.

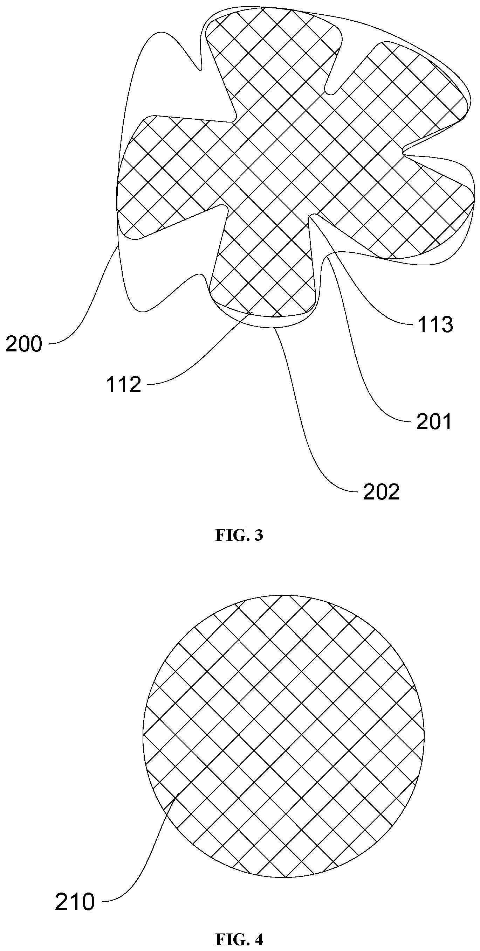

Further, referring to FIG. 3, FIG. 3 is a schematic diagram of the fixing portion, in a deformed state after implantation, of the occlusion device in Embodiment 1. Due to an irregular shape of a left atrial appendage wall, when the occlusion device 100 is entirely implanted, the entire fixing portion 110 expands radially, and outer sides of the edge areas 112 positioned on the outer side of the fixing portion 110 abut against the left atrial appendage wall. For this embodiment, the edge areas 112 project radially outward. Therefore, the edge areas 112 may be partially snapped into recesses 201 of the left atrial appendage wall 200 to increase contact between the edge areas 112 and the left atrial appendage. In addition, recesses 113 between adjacent edge areas 112 may also accommodate protrusions 202 of the left atrial appendage wall 200, and the adjacent edge areas 112 of the fixing portion 110 clamp the protrusions 202 of the left atrial appendage wall 200, thereby also increasing the contact between the edge areas 112 and the left atrial appendage, and entirely increasing radial support force of the fixing portion 110.

In addition, as the left atrial appendage wall 200 contracts inward in a rotating manner with cardiac contraction, new folds are generated, that is, new protrusions 202 and recesses 201 are generated, and the protrusions can be further adaptively snapped into the recesses 113 between the edge areas 112 and/or the outer sides of the edge areas 112 can be further adaptively snapped into the recesses, thereby greatly increasing the contact area between the occlusion device 100 and the left atrial appendage wall 200, and forming overall snap-fit through abutting at a plurality of positions. As a result, the occlusion device 100 in this embodiment has better radial support than a traditional design, that is, the occlusion device 100 is fitted more tightly with the left atrial appendage wall 200.

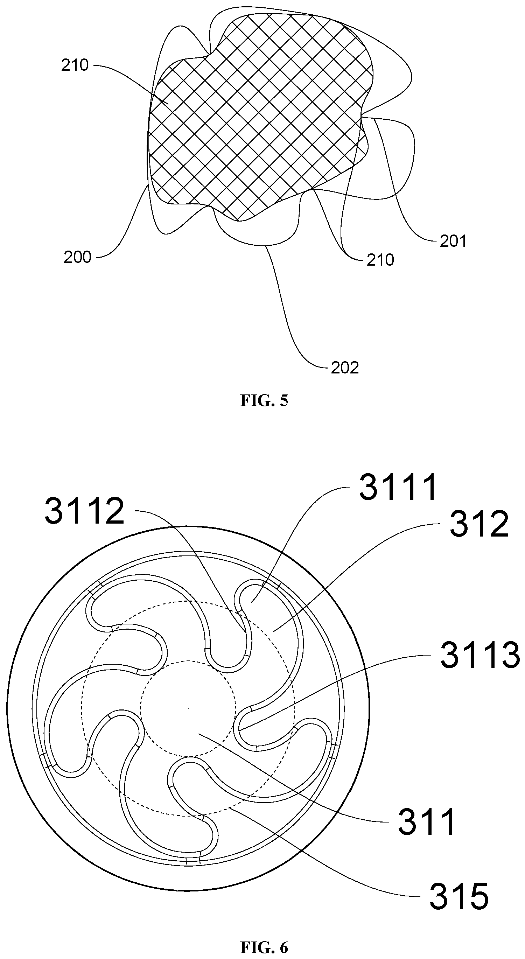

With reference to FIG. 4 to FIG. 5, the effect of this embodiment is further described. FIG. 4 is a top view of a fixing portion, from a distal end to a proximal end, of a traditional braided structure, and FIG. 5 is a schematic diagram of the fixing portion, in a deformed state after implantation, of the traditional braided structure. When the fixing portion 210 of the traditional braided structure is released on the left atrial appendage wall 200, just as mentioned above, the braided structure distributes concentrated stress to the periphery. For the braided structure itself, when the braided structure is stressed, deformation at a stress point is maximal, deformation at positions far from the stress point is gradually reduced, and a regional pit is formed on the whole. Accordingly, when the fixing portion 110 is implanted into the left atrial appendage and expands, the protrusions 202 of the left atrial appendage wall 200 compress the outer surface of the fixing portion 110, and thus peripheral areas of the compressed positions are sunken inward to form pits, while the recesses 201, positioned near the protrusions 202, of the left atrial appendage wall 200 are farther from the surface of the fixing portion 210. As a result, the contact between the traditional fixing portion 210 after implantation and the left atrial appendage wall 200 is point contact and surface contact, and an actual contact area is small and much smaller than the contact area of the fixing portion 110 in this embodiment. Since the morphology of the left atrial appendage wall 200 varies from person to person, this embodiment has a better effect when significant recesses 201 or protrusions 202 are present on the left atrial appendage wall.

It should be noted that the morphology of the left atrial appendage wall 200 in figures is only for illustrative, and actually, due to distribution of pectinate muscles on the left atrial appendage wall 200, the left atrial appendage wall 200 exhibits a higher density of the protrusions 202 and the recesses 201, whereby this embodiment significantly enhances an anchoring capability compared with the traditional braided structure with an approximately circular cross section along the axis. It should be further noted that an abutted state formed by the edge areas 112 and the recesses 201 of the left atrial appendage wall 200, and/or the recesses 113 and the protrusions 202 of the left atrial appendage wall 200 in this embodiment can also play a limiting role on the whole, the fixing portion 110 is prevented from rotating to avoid damage to or dislodgement on the left atrial appendage wall 200, and a plurality of mutually snapped areas are formed through abutting to enhance an anchoring capability of the fixing portion 110.

Therefore, under certain conditions, by adopting the solution of this embodiment, anchoring barbs or other sharp configurations that easily damage the left atrial appendage wall 200 can be omitted.

In this embodiment, proximal and distal edges of the fixing portion 110 are converged and closed, the proximal end of the fixing portion 110 is converged and connected to a connecting component 130, and the distal end of the fixing portion 110 is converged and connected to a steel sleeve.

In this embodiment, the surface of the proximal edge of the fixing portion 110 is located on a proximal side of the connecting component 130, and at the position of the connecting component 130, the fixing portion 110 is converged inward from the proximal end to the distal end. When the edge area 112 of the fixing portion 110 is stressed, force is transmitted to the vicinity of the connecting component 130, and stress on the connecting component 130 inclines toward the distal end, such that the connecting component 130 strains the sealing portion 120, to maintain an anchoring effect of the occlusion device 100.

In another embodiment, the distal edge of the fixing portion 110 is open, and the proximal end is closed entirely.

In another embodiment, the proximal edge of the fixing portion 110 is open, and the distal end is closed entirely.

In this embodiment, to achieve a good anchoring effect, a number of edge areas 112 of the fixing portion 110 is not less than 4, and preferably 6 in most cases, that is, the number of edge areas 112 is between 4 and 6.

### Embodiment 2

Objectively speaking, an increase in a number of edge areas of a fixing portion results in tighter fit with a left atrial appendage wall and a corresponding increase in a number of recesses, and an increase in depth of the recess of the fixing portion, that is, an increase in a distance from the recess to an outer side of an edge area results in more left atrial appendage morphologies that can be accommodated. Based on the above design principles, Embodiment 1 tends to employ more edge areas and deeper recesses. However, such configuration simultaneously leads to an increase of an overall surface area, reduced adaptability to the contraction of the left atrial appendage, and a significant increase in the difficulty of retraction in a sheath. A difference between this embodiment and Embodiment 1 lies in that a shape of a fixing portion is further improved.

Therefore, this embodiment provides a fixing portion in a special shape. Referring to FIG. 6, FIG. 6 is a top view of an occlusion device from a distal end to a proximal end in Embodiment 2 of the present disclosure, and details are as follows.

In this embodiment, a cross-sectional shape of a central area 311 of the fixing portion 310 remains approximately circular, and a plurality of edge areas 312 extend outward from a base in a clockwise or counterclockwise direction, such that the edge areas 312 of the entire fixing portion 310 are oblique and radial on an axial cross section. When the fixing portion 310 retracts in the sheath, the edge areas 312 can be overlapped and accommodated; and compared with a configuration that the edge areas do not incline in the same direction, the design of this embodiment may allow the edge areas 312 to be overlapped in a preset inclination direction to achieve a smaller volume.

Therefore, since shapes of the edge areas 312 are not specified, to ensure that the edge areas 312 are overlapped in the same direction during retraction in the sheath, this embodiment further specifies inclination. The edge areas 312 include wing portions 3111 extending outward, and two adjacent side faces 3112 are positioned between adjacent wing portions 3111, and on the axial cross section of the fixing portion 310, curvatures of the two side faces 3112 between the adjacent wing portions 3111 are equally positive or negative, that is, the two side faces 3112 between the adjacent wing portions 3111 extend obliquely in substantially the same direction.

In the same way, since the shapes of the edge areas 312 are not specified, recesses 3113 may be formed between adjacent wing portions 3111; and the recesses 3113 or other shape changes cause their radial bottoms to be closer to or farther from an axis, such that a boundary of the central area 311 becomes blurred. In this embodiment, the boundary of the central area 311 is limited by the recesses 3113, that is, the central area 311 is a cylindrical area formed by taking the axis as a center and taking a shortest distance among distances from all recesses 3113 to the axis as a radius. In other words, the central area 311 is a cylinder formed by taking the axis as a center and taking a position, closest to the axis, of the fixing portion 110 as a separation point. Based on this, areas on an outer side of the central area 311 are all the edge areas 312, the wing portions 3111 refer to portions significantly protruding in the edge areas 312, and a single wing portion 3111 corresponds to a single edge area 312.

In addition to having good sheath entry flexibility, after completion of implantation, with the volume of the left atrial appendage contracted to a smaller size with the contraction of the heart, and based on a contraction process of the heart, the design of this embodiment also allows the fixing portion 310 to reduce to a small volume, thereby adapting to left atrial appendages in different sizes. This prevents a phenomenon that adjacent edge areas 312 approach each other from opposite directions and are abutted against and interfered with each other to hinder contraction, and local stress is concentrated to damage the left atrial appendage.

In another embodiment, a cross-sectional shape of a central area 311 of the fixing portion 310 is approximately circular, and from a distal end to a proximal end, a plurality of edge areas 312 extend outward from a base in a clockwise direction.

In another embodiment, a cross-sectional shape of a central area 311 of the fixing portion 310 remains approximately circular, and from a distal end to a proximal end, a plurality of edge areas 312 extend outward from a based in a counterclockwise direction.

For this embodiment, it should also be noted that significant gaps are formed between the wing portions 3111, to reserve more deformation allowance, thereby facilitating retraction of the entire fixing portion 310 in the sheath. Therefore, to ensure that the fixing portion smoothly enters the sheath, on a cross section of the fixing portion 310, a radial center line 315 of the edge area 312 is defined as an imaginary circle that passes through a midpoint of the edge area 312 in a radial direction, and by taking the radial center line 315 of the edge area 312 as a dividing line, on a side of the edge area 312 close to the axis (i.e., an inner side) along the radial center line 315, a maximum separation distance between the adjacent wing portions 3111 is less than a maximum circumferential width of the two adjacent wing portions 3111 themselves. The design of the wing portions 3111, which satisfy such conditions, is referred to as a spaced design. Accordingly, in the spaced design, when a single wing portion 3111 is subjected to significant compression and deformed, the single wing portion 3111 can span across a gap with an adjacent wing portion and is supported by the adjacent wing portion 3111, to ensure support strength of the entire fixing portion 310.

In another embodiment, further, on the side of the edge area 312 close to the axis (i.e., the inner side) along the radial center line 315, a maximum separation distance between the adjacent wing portions 3111 is less than a maximum circumferential width of any wing portion 3111 in all the wing portions 3111.

It should be noted that this effect can be achieved only when significant wing portions 3111 are formed on the fixing portion 310 and significant gaps are formed between adjacent wing portions 3111. In this embodiment, from the perspective of the entire fixing portion 310, a wing portion 3111 is considered as the significant wing portion 3111 described above when its radial extension distance exceeds at least one third of the radius of the entire fixing portion 310. In case of insignificant wing portions, after deformation of a wing portion, the deformation is directly transmitted to a base of the wing portion, and the wing portion itself is not abutted against and supported by an adjacent wing portion based on the deformation of the wing portion, that is, the wing portion stressed directly cannot deform nor span across a gap to be supported by the adjacent wing portion 3111. In summary, the design of the fixing portion 310 has the above-mentioned support effect only after the significant wing portions 3111 are arranged.

### Embodiment 3

In this embodiment, based on a risk control for an implant, to further enhance anti-dislodgement performance, an anchoring component 410 may be additionally arranged on a wing portion 3111.

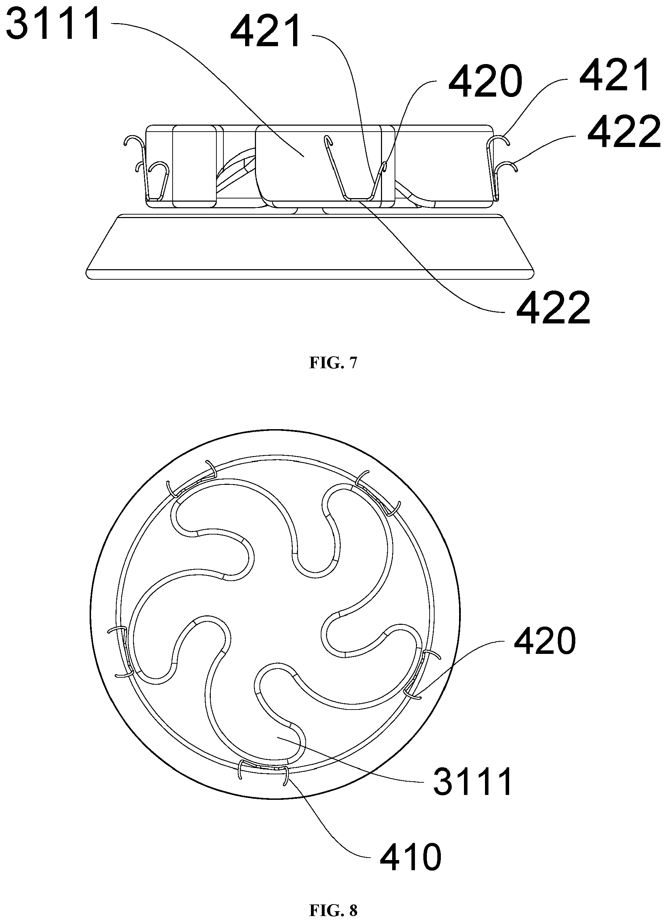

Referring to FIG. 7 to FIG. 8, FIG. 7 is a schematic structural diagram of a fixing portion of an occlusion device in Embodiment 3 of the present disclosure, and FIG. 8 is a top view of the fixing portion, from a distal end to a proximal end, of the occlusion device in Embodiment 3 of the present disclosure.

In this embodiment, the anchoring component 410 is not a part of a fixing portion 310, that is, the anchoring component 410 is not formed directly on a main body of the fixing portion 310 or by extending a part of the main body, and is not rigidly secured to the fixing portion 310 through welding and the like. In this embodiment, the anchoring component 410 is secured to the fixing portion 310 in a manner of binding through a flexible component (such as a suture). In this embodiment, the anchoring component 410 is secured to an outer side of the wing portion 3111, and a free end of the anchoring component 410 is exposed and extends obliquely, to pierce a preset position to achieve anchoring.

In another embodiment, a hook 420 of the anchoring component 410 passes through a framework of the wing portion 3111 from an inner side to the outer side, and the free end of the anchoring component 410 is exposed and extends obliquely, to pierce a preset position to achieve anchoring.

In this embodiment, the anchoring component 410 includes hooks 420 arranged in pairs, which are a hook 411 and a hook 412 respectively. In general, the hooks 420 extend from rods 421 (additional hooks may also be secured to the rods 421). Therefore, the anchoring component 410 further includes the rods 421 arranged in pairs, and a group of rods 421 are connected through at least one rod 422. It should be noted that in addition to having a basic connecting function, the rod 422 can also distribute force applied to the pair of hooks 420. This not only prevents the hooks 420 from exerting excessive stress on an implant site, thereby avoiding an unduly large wound, but also creates a mutual pulling effect between the two hooks 420 as the stress is shared between the two hooks at different locations. This results in a pincer-like clamping effect. The anchoring component 410 with such configuration achieves a better anchoring effect without changing a piercing depth.

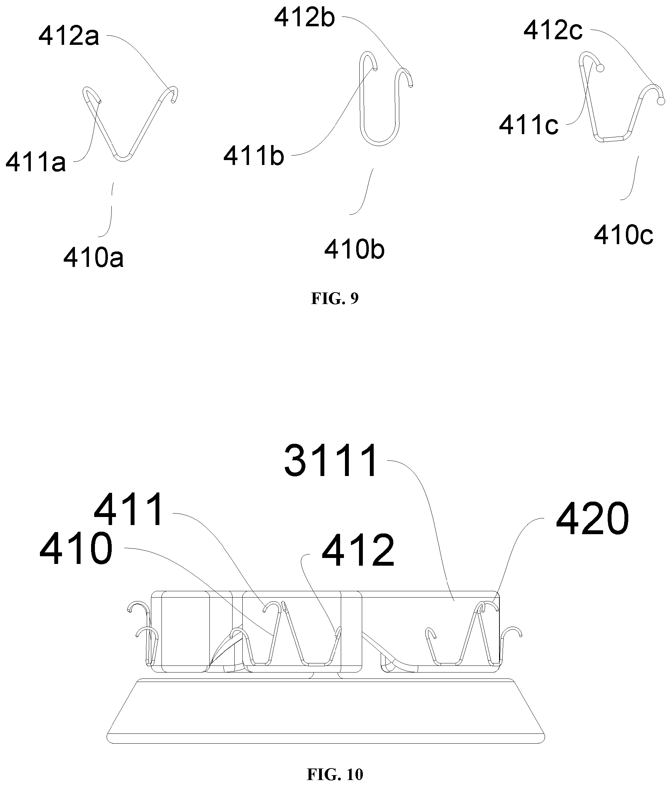

Referring to FIG. 9, FIG. 9 is a schematic structural diagram of a plurality of optional anchoring components in Embodiment 3 of the present disclosure. In this embodiment, a plurality of anchoring components 410 may be selected or combined. Under normal conditions, an anchoring component 410a that is distributed symmetrically and arranged substantially in a "V" shape may be adopted, and in this design, a first hook 411a and a second hook 412a of the anchoring component 410a are located at the same axial position of the fixing portion of the occlusion device; a design that a first hook 411b and a second hook 412b of an anchoring component 410b are located at different heights, as shown in the figure, may also be adopted, in this design, the first hook 411b and the second hook 412b of the anchoring component 410b are located at different axial positions of the fixing portion of the occlusion device, and the first hook 411b is located on a distal side of the second hook 412b; and a design that a first hook 411c and a second hook 412c of an anchoring component 410c are different in height and extension direction, as shown in the figure, may also be adopted, in this design, the first hook 411c and the second hook 412c of the anchoring component 410c are located at different axial positions of the fixing portion of the occlusion device, the first hook 411c is located on a distal side of the second hook 412c, and extension directions of the first hook 411c and the second hook 412c are different.

On the basis of this embodiment, when the extension directions of the first hook 411 and the second hook 412 are different, the anchoring component 410 is in contact with a to-be-anchored position in a plurality of directions, to achieve more stable anchoring. It should be noted that this function can be achieved only when the anchoring component 410 is arranged at a peripheral position of the wing portion 3111, because the wing portion 3111 itself can extend into interiors of pectinate muscles. A fixing portion of a traditional occlusion device has a complete circumferential surface, and cannot well extend into and fit the interiors of the pectinate muscles, such that the fixing portion of the traditional occlusion device is always in direct contact with outer surfaces of the pectinate muscles. Because the fixing portion cannot extend into or be snapped into the interiors of the pectinate muscles, a corresponding anchoring component of the traditional occlusion device can only be arranged in a specific direction. Theoretically, if anchoring components of the traditional occlusion device are directly arranged in different directions, because the fixing portion, after release, of the traditional occlusion device expands substantially in a circumferential direction to tightly fit partial pectinate muscles, the anchoring components arranged not in the circumferential direction cannot pierce well, that is, the fixing portion of the traditional occlusion device adopts the design of the hooks 420 in different extension directions in this embodiment and cannot achieve a better anchoring effect.

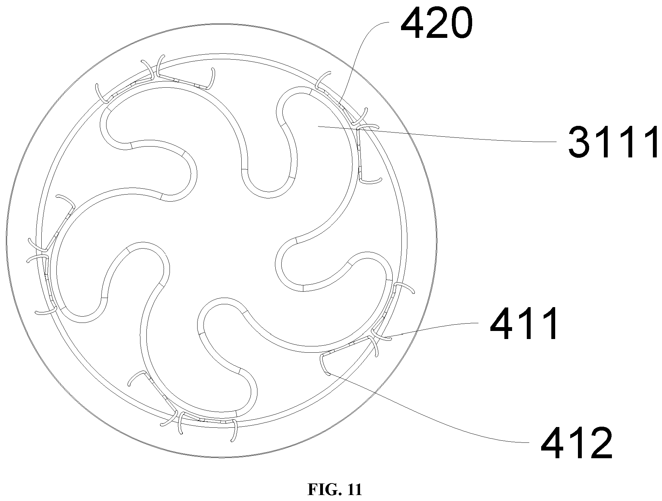

In another embodiment, referring to FIG. 10 to FIG. 11, FIG. 10 is a schematic structural diagram of an occlusion device in this embodiment, and FIG. 11 is a top view of the occlusion device from a distal end to a proximal end in this embodiment. At least two anchoring components 410 are arranged on a wing portion 3111, and the two anchoring components 410 are not completely overlapped in a circumferential direction, such that the two anchoring components may complement each other, that is, a single wing portion 3111 is provided with hooks 420 at a plurality of positions, to ensure that each wing portion 3111 can achieve anchoring. In this embodiment, it should also be noted that an outer edge of the wing portion 3111 gradually converges toward an axis, and adjacent anchoring components 410 are not on the same circumferential surface on the whole, to further achieve the hooks 420 of the anchoring components 410.

Preferably, on the same wing portion 3111, for two adjacent anchoring components 410, a first hook 411 is located on a distal side of a second hook 412, and the first hook 411 is adjacent to a first hook of an adjacent anchoring component 410, such that areas of the entire fixing portion, which play an anchoring role, are distributed at different heights, to achieve a better anchoring effect.

It should be noted that the technical features of the above embodiments can be combined freely, and can also be applied to various occlusion devices mentioned above and those with similar structures. To make descriptions concise, not all possible combinations of the technical features of the above embodiments are described. However, these combinations of the technical features should fall within the scope of the present disclosure as long as they are not contradictory.

It should also be noted that the above embodiments do not preclude the technical solutions of additionally arranging anchoring barbs. To meet specific requirements, the anchoring barbs may be arranged in the above embodiments as needed.

The above embodiments only describe some implementations of the present disclosure specifically and in detail, but cannot be construed as a limitation to the patent scope of the present disclosure. It should be noted that those of ordinary skill in the art can further make several transformations and improvements without departing from the concept of the present disclosure. These transformations and improvements all fall within the protection scope of the present disclosure. Therefore, the protection scope of the present disclosure should be subject to the accompanying claims.

## Claims

1. An occlusion device, comprising a fixing portion and a sealing portion, wherein the fixing portion comprises a mesh braided structure, the fixing portion comprises a central area and a plurality of edge areas distributed around the central area in a circumferential direction and extending outward in a radial direction, and a recess is formed between two adjacent edge areas.

2. The occlusion device according to claim 1, wherein a proximal end and a distal end of the fixing portion are converged and closed.

3. The occlusion device according to claim 1, wherein the edge areas comprise wing portions extending outward, and two adjacent side faces between adjacent wing portions extend obliquely in the same direction on the same cross section.

4. The occlusion device according to claim 1, wherein a number of edge areas of the fixing portion is between 4 and 6.

5. The occlusion device according to claim 1, wherein the plurality of edge areas of the fixing portion are oblique and radial on an axial cross section.

6. The occlusion device according to claim 1, wherein the plurality of edge areas of the fixing portion extend outward from a base in a clockwise direction, or the plurality of edge areas extend outward from a base in a counterclockwise direction.

7. The occlusion device according to claim 3, wherein with a radial center line of the edge area as a dividing line, on an inner side of the edge area along the radial center line, a maximum separation distance between the adjacent wing portions is smaller than a maximum circumferential width of the adjacent wing portions.

8. The occlusion device according to claim 7, wherein with the radial center line of the edge area as the dividing line, on the inner side of the edge area along the radial center line, the maximum separation distance between the adjacent wing portions is smaller than a maximum circumferential width of any wing portion.

9. The occlusion device according to claim 1, wherein an anchoring component is arranged on the fixing portion, the anchoring component is bound to the fixing portion through a flexible component, and a free end of the anchoring component is exposed.

10. The occlusion device according to claim 9, wherein the anchoring component comprises a first hook and a second hook which are connected to each other, and the first hook is positioned on a distal side of the second hook.
